# EUROPEAN PATENT APPLICATION

(11) **EP 0 522 433 A1**
(43) Date of publication of application: **13.01.1993**
(21) Application number: 92111140.7
(22) Date of filing: 01.07.1992
(51) Int. Cl.: A61K 31/12, A61K 9/08, A61K 47/44

(54) **Oral aqueous solutions containing ubidecarenone**

(30) Priority: 11.07.1991 IT MI911919
(71) Applicant: INVERNI DELLA BEFFA S.P.A., I-20141 Milano (IT)
(72) Inventor: Seghizzi, Roberto, I-20141 Milano (IT); Furiosi, Giuseppe, I-20141 Milano (IT); Martinelli, Ernesto Marco, I-20141 Milano (IT); Pifferi, Giorgio, I-20141 Milano (IT)
(74) Representative: Minoja, Fabrizio

(57) **Abstract**

Pharmaceutical formulations, in the form of oral aqueous solutions, containing ubidecarenone as active ingredient, characterized in that they contain polyethoxylated 40 hydrogenated castor oil, as non ionic surfactant.

## Description

The present invention relates to pharmaceutical formulations, in the form of oral aqueous solutions, containing ubidecarenone as active ingredient.

Ubidecarenone, or coenzyme Q10, is a physiological compound, which is present in the cells as a component of the mitochondrial respiratory chain (De Pierre V.C. et al., Ann. Rev. Biochem., 46, 201, 1977; Nakamura T. et al., Chem. Pharm. Bull. 27, 1101, 1979). Ubidecarenone acts directly on oxidative phosphorylation processes for the energy production (ATP formation) through metabolic pathways, especially the aerobic ones.

Ubidecarenone requirement can increase in normal subjects which undergo severe physical work, or in subjects suffering from cardiovascular diseases, chronic weakening diseases or subjected to chronic pharmacological treatments. Particularly, ubidecarenone deficit has been reported in ischemic cardiopathy, senile myocardiosclerosis and in hypertensive cardiopathy (Yamagami T. et al., Biomedical and Clinical Aspects of Coenzime Q10, Elsevier/North-Holland Biomedical Press Vol. 3, 79, 1981; Kishi T. et al., ibidem Vol. 3, 67). In the above pathologies, exogenous administration of ubidecarenone gives rise to remarkable improvements and to important therapeutic results (Yamasawa G., Biomedical and Clinical Aspects of Coenzime Q10, Elsevier/North-Holland Biomedical Press Vol. 2, 333, 1980).

Ubidecarenone is a lipophilic solid, having low melting point, and is practically water-insoluble. These characteristics limit the possibility to prepare stable aqueous solutions, particularly in the case of formulations for oral use, with the purpose to achieve a bioavailability higher than the one obtainable with conventional solid formulations (tablets, capsules, extemporary suspensions).

US 3,113,073 and BE 906034 patents disclose oral, parenteral and topical aqueous formulations containing ubidecarenone, which has been solubilized in a mixture of polyethoxylated castor oil and a water-soluble amide, such as N,N-dimethylacetamide. However, these amides can not be used as solubilizing agents in pharmaceutical formulations owing to their well-known embryotoxic and teratogenic effects (Merkle J. and Zeller H., Arzneim. Forsch. 30, 1557, 1980; von Kreybig T. et al. ibid 19, 1073, 1969; Stula E.F. e Krauss W.C. Toxic. appl. Pharmac. 41, 35, 1977).

It has now been found, and is an object of the present invention, that ubidecarenone stable aqueous solutions for the oral use can be prepared without using amides.

According to the invention, the formulations contain polyethoxylated 40 hydrogenated castor oil, a non-ionic surfactant, acceptable for the oral use, which acts as solubilizing agent of the active ingredient.

It has further been found that a polyalcohol, such as preferably sorbitol up to 25% (w/v), not only acts normally as thickening and sweetening agent, but also as stabilizing agent of the solution.

The non ionic surfactant, contained in amounts equal to about 10 times the weight of the active ingredient, can be reduced till 3 times the weight of the active ingredient when in the presence of lecithins or other non ionic surfactants beloging to the class of polyoxyethylenesorbitane esters of fatty acids, used in amount equal to the weight of the active ingredient. Further, it has been found that the use of polyethoxylated 35 castor oil, containing unsaturated acyl residues, is not suitable for the preparation of stable solutions.

The formulations of the invention can further contain pH regulating agents, chelating agents, sweetening agents, preservatives and ethyl alcohol.

The ubidecarenone solutions, obtained according to the invention, proved to be stable even in particular storage conditions, such as for example those characterized by thermal stress.

Said tests (S.S. Davis in: Stability Testing of Drug Products, W. Grimm and K. Tomae, Eds, Wissenschaftliche Verlagsgesellschaft GmbH, Stuttgart 1987) are recommended in order to stress out pharmaceutical preparation potential instability, which can appear during storage and shipping. Particularly, Food and Drug Administration "Draft Guideline for Stability Studies for Human Drugs and Biologics" recommends to subject pharmaceutical preparations to temperature cycles, as to simulate the fluctuations which can happen once the product is in distribution channels.

A peculiar aspect of the present invention is that the compositions of the invention allow to obtain blood levels which are higher and more prolonged than the ones obtained with the conventional formulations.

The following Examples further illustrate the invention.

### Example 1

| | |
|---|---|
| Ubidecarenone | 50 mg |
| Polyethoxylated 40 hydrogenated castor oil | 500 mg |
| Non crystallizing (70%) sorbitol solution | 2500 mg |
| Citric acid monohydrate | 5 mg |
| Sodium edetate | 5 mg |
| Sodium benzoate | 20 mg |
| Sodium saccharin | 2 mg |
| Flavouring agents | 16 mg |
| Water q.s. to | 10 ml |

The preparation of the above formulation comprises heating the ubidecarenone and polyethoxylated 40 hydrogenated castor oil mixture up to 70°C under constant stirring until complete solubilization of the active ingredient is achieved. All the other excipients but the flavouring agents are dissolved in water at 70°C and the solution is added, while stirring, to the active ingredient solution.

The resulting mixture is cooled to 40°C and the flavouring agents are finally added.

### Example 2

| | |
|---|---|
| Ubidecarenone | 50 mg |
| Polyethoxylated 40 hydrogenated castor oil | 150 mg |
| Non crystallizing (70%) sorbitol solution | 2500 mg |
| Polysorbate 80 | 50 mg |
| Sodium benzoate | 20 mg |
| Citric acid monohydrate | 5 mg |
| Sodium saccharin | 2 mg |
| Flavouring agents | 20 mg |
| Water q.s. to | 10 ml |

Formulation prepared according to the procedure of Example 1.

### Example 3

| | |
|---|---|
| Ubidecarenone | 50 mg |
| Polyethoxylated 40 hydrogenated castor oil | 150 mg |
| Non crystallizing (70%) sorbitol solution | 1500 mg |
| Alcohol | 500 mg |
| Phosphatidylcholine | 50 mg |
| Sodium benzoate | 20 mg |
| Citric acid monohydrate | 5 mg |
| Sodium saccharin | 3 mg |
| Flavouring agents | 20 mg |
| Water q.s. to | 10 ml |

Formulation prepared according to the procedure of Example 1; phosphatidylcholine is dissolved in alcohol at room temperature, then is added before flavouring agents.

The Examples of further 3 formulations, used as references in comparative stability trials with compositions of Examples 1-3 of the present invention, are also reported.

### Example 4

Corresponding to Example 1, but using polyethoxylated 35 castor oil instead of polyethoxylated 40 hydrogenated castor oil.

### Example 5 (Corresponding to Example 1, but without sorbitol).

### Example 6 (Corresponding to Example 1 of BE 906034).

| | |
|---|---|
| Ubidecarenone | 100 mg |
| Polyethoxylated 35 castor oil (Cremophor EL^{R}) | 800 mg |
| 70% Sorbitol | 0,4 ml |
| N,N-Dimethylacetamide | 0,2 ml |
| Water | 1,6 ml |

Physical stability of the above described formulations was tested according to the following methods.

Test A - the formulations are subjected to a maximum of 15 freeze-thaw cycles (24 hours at -20°C and 24 hours at room temperature) (cycling test); the test is stopped when a precipitate is detected on visual inspection;
Test B - the formulations are stored at room temperature for 3 months;
Test C - the compositions are stored at 37°C for 3 months.

The test results, reported in Table 1, show the higher stability of the formulations of the invention.

In order to evaluate ubidecarenone absorption from the new oral formulations in comparison with a marketed preparation (ubidecarenone 50 mg, extemporary suspension), active ingredient plasma levels were evaluated after "cross-over" administration of the two compositions to a same subject. The study was carried out on 3 healthy volunteers aged from 25 to 35 (two of them female and one male). Firstly, each subject was administered with an oral dose of 2 x 50 mg of ubidecarenone (50 mg single dose vial, extemporary suspension), and, subsequently, after 1 week-wash-out, an oral dose of 2 x 50 mg of the ubidecarenone solution prepared as in Example 1.

Blood samples (5 ml) were withdrawn at time 0 (before administration) and 1, 2, 4, 6, 8, 10 and 24 hours after the administration and were put into test tubes containing sodium citrate as anticoagulant. The blood samples were put immediately after the withdrawal at 0°C and centrifuged (20' at 2500 rpm) within 10' from withdrawal to separate plasma. Plasma was added with 0.2 ml of a solution containing 0.1 mg of butylhydroxytoluene (BHT) in 1 ml of ethanol, as antioxidant agent.

Ubidecarenone plasma levels were determined by means of a specific HPLC method, using a reverse-phase column, following extraction with n-hexane.

The obtained results (Table 2) shows the higher bioavailability of the formulations of the present invention in comparison with the marketed extemporary suspension.

**Table 1**

| Physical stability of ubidecarenone formulations. | | | | |
|---|---|---|---|---|
| Formulations | Time "0" | Aspect | | |
| | | Test A | Test B | Test C |
| Example 1 | clear solution | 15 | unch. | unch. |
| Example 2 | clear solution | 10 | unch. | unch. |
| Example 3 | clear solution | 12 | unch. | unch. |
| Example 4 | clear solution | 3 | unch. | prec. |
| Example 5 | clear solution | 2 | unch. | unch. |
| Example 6 | clear solution | 1 | - | - |
| Test A = cycling test (24 hours at -20°C, 24 hours at room temperature); number of cycles passed Test B = room temperature, 3 months Test C = 37°C, 3 months unch. = unchanged prec. = precipitated. | | | | |

**Table 2**

| Pharmacokinetic parameters following administration of two oral ubidecarenone formulations to healthy volunteers (Mean of 3 values). | | | | |
|---|---|---|---|---|
| Formulations | single dose (mg) | AUC (0-24) (ug.h.ml) | Tmax (h) | Cmax (ug.ml⁻¹) |
| Solution (Example 1) | 2 x 50 | 6.16 | 10 | 0.35 |
| Extemporary* suspension | 2 x 50 | 2.38 | 7 | 0.23 |

| | | | | |
|---|---|---|---|---|
| *Ubidecarenone 50 mg, lactose 87 mg, polyvinylpyrrolidone 10 mg, polisorbate 80 6 mg, sorbitol 800 mg, glycerine 400 mg, methyl p-hydroxybenzoate 15 mg, propyl p-hydroxybenzoate 3 mg, orange flavour 3 mg, water q.s. to 8 ml. | | | | |

## Claims

1. Pharmaceutical formulations, in the form of oral aqueous solutions, containing ubidecarenone as active ingredient, characterized in that they contain polyethoxylated 40 hydrogenated castor oil, as non ionic surfactant.

2. Pharmaceutical formulations according to claim 1, wherein polyethoxylated 40 hydrogenated castor oil is present in amounts equivalent to about 10 times the weight of the active ingredient.

3. Pharmaceutical formulations according to claim 1 or 2, further containing a polyalcohol.

4. Pharmaceutical formulations according to claim 3, wherein sorbitol is the polyalcohol.

5. Pharmaceutical formulations according to claims 3 or 4, wherein the polyalcohol is contained in amounts up to 25% w/v.

6. Pharmaceutical formulations according to claims 1 and 3, further containing lecithins or polyoxyethylenesorbitan esters in amount equal to the weight of the active ingredient.

7. Pharmaceutical formulations according to claim 6, wherein polyethoxylated 40 hydrogenated castor oil is present in amount equivalent to about 3 times the weight of the active ingredient.

8. Pharmaceutical formulations according to any one of the preceding claims, further containing pH regulating agents, chelating agents, sweetening agents, preservatives and ethyl alcohol.

9. Pharmaceutical formulations according to any one of the preceding claims, characterized in that they contain ubidecarenone in amounts from 0.1 to 1%.

10. A process for the preparation of the pharmaceutical formulations of claims 1-8 which comprises solubilizing ubidecarenone into polyethoxylated 40 hydrogenated castor oil and subsequently adding an aqueous solution of the remaining excipients and optionally adding flavouring agents.

11. The use of polyethoxylated 40 hydrogenated castor oil for the preparation of oral pharmaceutical ubidecarenone formulations which are stable and capable of providing higher and more prolonged blood levels.
